# FASCICULE DE BREVET EUROPEEN

(11) **EP 1 115 354 B1**
(45) Date de publication et mention de la délivrance du brevet: **26.11.2003**
(21) Numéro de dépôt: 99929409.3
(22) Date de dépôt: 06.07.1999
(51) Int. Cl.: A61F 2/40

(54) **DISPOSITIF POUR LA FIXATION D'UN INSERT EN MATIERE PLASTIQUE**
VORRICHTUNG ZUR BEFESTIGUNG EINES EINSATZES AUS KUNSTSTOFF
DEVICE FOR FIXING A PLASTIC MATERIAL INSERT

(30) Priorité: 06.07.1998 FR 9808778
(43) Date de publication de la demande: 18.07.2001
(73) Titulaire: Tornier SA, 38330 Saint Ismier (FR)
(72) Inventeur: TORNIER, Alain, F-38330 Saint Ismier (FR); OUDARD, Jean-Loup, F-38330 Saint Nazaire lès Eymes (FR)
(74) Mandataire: Schmitt, John
(86) Numéro de dépôt international: FR9901624
(87) Numéro de publication internationale: WO00001326

(56) Documents cités:
- EP-A- 0 809 986
- FR-A- 2 683 142
- FR-A- 2 695 313
- FR-A- 2 718 635
- FR-A- 2 741 796
- US-A- 4 550 450
- US-A- 4 964 865
- US-A- 5 108 442
- US-A- 5 514 183

## Description

La présente invention est relative à un dispositif pour la fixation d'un insert en matière plastique sur une embase métallique d'une prothèse, et notamment d'une prothèse glénoïdienne de l'omoplate.

On connaît d'après le brevet français n° 2 741 796 au nom du demandeur, une prothèse glénoïdienne de l'omoplate comportant un dispositif de fixation de l'insert en matière plastique sur l'embase métallique.

L'embase métallique comprend une cuvette qui est délimitée par un rebord possédant deux tronçons parallèles. Chaque tronçon parallèle s'étend verticalement à partir de la face horizontale de l'embase métallique. De plus les tronçons présentent chacun des nervures orientées vers l'intérieur de la cuvette.

L'insert en matière plastique comprend une surface articulaire dont le contour est identique à celui de l'embase métallique. L'insert comporte à l'opposé de la surface articulaire une partie en saillie de même contour que la cuvette afin d'être engagée dans cette dernière.

Une lèvre est prévue parallèle à l'un des bords de la partie en saillie pour permettre l'encliquetage de l'insert à l'intérieur et entre les tronçons parallèles de la cuvette. La lèvre est obtenue grâce à une rainure ménagée dans la partie en saillie de l'insert.

Le brevet français n° 2 741 796 au nom du demandeur décrit également une variante du dispositif de fixation de l'insert sur l'embase qui est symétriquement l'opposé de celui décrit ci-dessus. En effet, l'embase métallique comporte à l'intérieur de la cuvette une lèvre souple qui coopère avec le profil complémentaire de la partie en saillie de l'insert en matière plastique.

On constate que les dispositifs de fixation décrits dans le brevet français n° 2 741 796 comportent certains inconvénients en ce qui concerne la retenue de l'insert en matière plastique à l'intérieur de la cuvette de l'embase métallique lorsque la prothèse est soumise à des efforts de fonctionnement qui sont importants.

En effet, la prothèse est soumise, par exemple, à des contraintes d'arrachement dues à la réduction de la luxation, qui séparent l'insert en matière plastique de l'embase métallique.

C'est à ces inconvénients qu'entend plus particulièrement remédier la présente invention.

Le dispositif de fixation suivant la présente invention à pour objet de proposer un système d'assemblage qui résiste aux différentes contraintes de fonctionnement de l'articulation et qui est facile à mettre en place sur le patient.

Le dispositif de fixation suivant la présente invention comprend :
- Une embase métallique pourvue d'une cuvette qui est délimitée par un rebord périphérique en forme de V possédant des tronçons opposés et inclinés qui sont reliés par une portion courbe, et par un bord périphérique solidaire d'une butée positionnée du côté de la base la plus ouverte du V du rebord,
- Et un insert en matière plastique qui comporte à l'opposé de sa surface articulaire une partie en saillie présentant un profil complémentaire à celui de la cuvette pour coopérer avec les tronçons inclinés et venir en appui contre la butée.

Le dispositif de fixation suivant l'invention présente de préférence une embase métallique dont les tronçons inclinés comportent un profil en forme de queue d'aronde.

Le dispositif de fixation suivant l'invention comporte de préférence une butée qui s'étend verticalement à partir du plateau de l'embase métallique.

Le dispositif de fixation suivant l'invention présente de préférence une butée qui comporte une portion droite délimitée à chaque extrémité par une portion courbe.

Le dispositif de fixation suivant l'invention présente de préférence un insert en matière plastique dont la partie en saillie comporte des bords périphériques opposés qui sont inclinés pour coopérer respectivement avec les tronçons du rebord périphérique en forme de V de l'embase métallique.

Le dispositif de fixation suivant l'invention présente de préférence un insert en matière plastique dont les bords périphériques opposés de la partie en saillie sont en forme de queue d'aronde afin de coopérer avec le profil des tronçons inclinés de la cuvette.

Le dispositif de fixation suivant l'invention comporte de préférence un insert en matière plastique pourvu d'une échancrure qui présente un profil complémentaire à celui du bord périphérique et de la butée.

Le dispositif de fixation suivant l'invention comporte de préférence un insert en matière plastique dont la partie en saillie comporte du côté de l'échancrure une portion droite se terminant à chaque extrémité par une portion courbe.

Egalement, la présente invention concerne une prothèse d'épaule comportant un dispositif de fixation d'un insert en matière plastique sur une embase métallique pourvue d'une cuvette qui est délimitée par un rebord périphérique en forme de V possédant des tronçons opposés et inclinés qui sont reliés par une portion courbe, et par un bord périphérique solidaire d'une butée positionné du côté de la base la plus ouverte du V du rebord, tandis que l'insert comporte à l'opposé de sa surface articulaire une partie en saillie présentant un profil complémentaire à celui de la cuvette pour coopérer avec les tronçons inclinés et venir en appui contre la butée.

La prothèse d'épaule suivant l'invention présente de préférence une embase métallique dont le rebord périphérique comprend des tronçons inclinés comportant un profil en forme de queue d'aronde.

Le dispositif de fixation suivant l'invention comporte de préférence une butée qui s'étend à partir du plateau de l'embase métallique et sur toute la hauteur du bord périphérique.

Le dispositif de fixation suivant l'invention présente de préférence une butée qui comporte une portion droite délimitée à chaque extrémité par une portion courbe.

La prothèse d'épaule suivant l'invention présente de préférence un insert en matière plastique dont la partie en saillie comporte des bords périphériques opposés qui sont inclinés pour coopérer respectivement avec les tronçons du rebord périphérique en forme de V de l'embase métallique.

La prothèse d'épaule suivant l'invention présente de préférence un insert en matière plastique dont les bords périphériques opposés de la partie en saillie sont en forme de queue d'aronde afin de coopérer avec le profil des tronçons inclinés.

La prothèse d'épaule suivant l'invention présente de préférence un insert en matière plastique dont la partie en saillie comporte une échancrure qui présente un profil complémentaire à celui du bord périphérique et de la butée.

La prothèse d'épaule suivant l'invention présente de préférence un insert en matière plastique dont la partie en saillie comporte du côté de l'échancrure une portion droite se terminant à chaque extrémité par une portion courbe.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective illustrant le dispositif de fixation prévu sur l'embase métallique suivant la présente invention.
Figure 2 est une coupe montrant la cuvette de l'embase métallique suivant la présente invention.
Figure 3 est une vue de dessus représentant le profil en forme de V de la cuvette de l'embase métallique suivant la présente invention.
Figures 4a et 4b sont des vues montrant l'insert en matière plastique suivant la présente invention.
Figures 5 et 6 sont des coupes illustrant l'insert en matière plastique monter sur l'embase métallique.

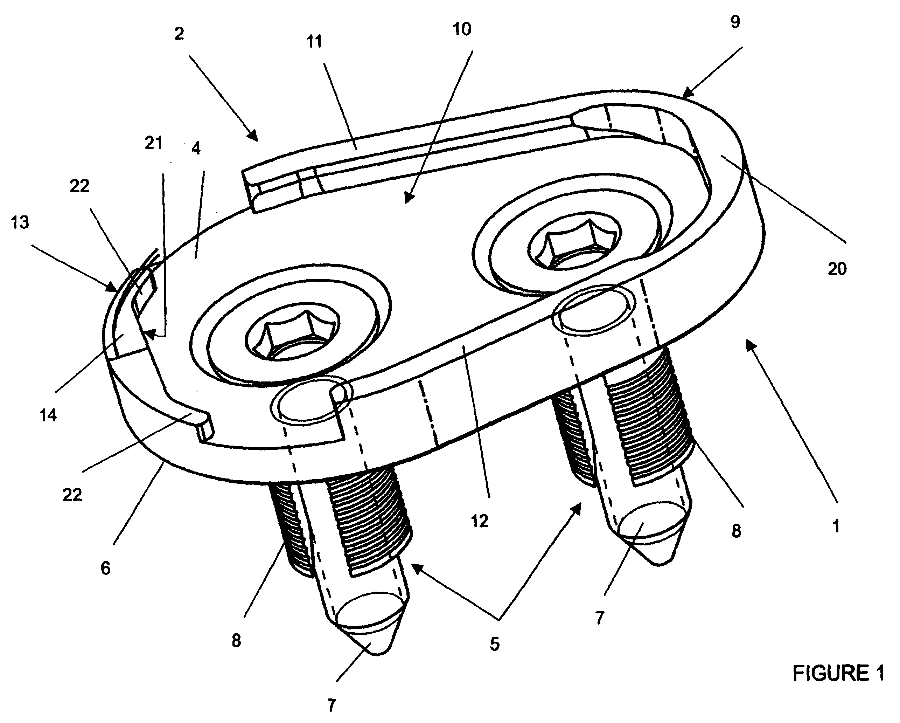

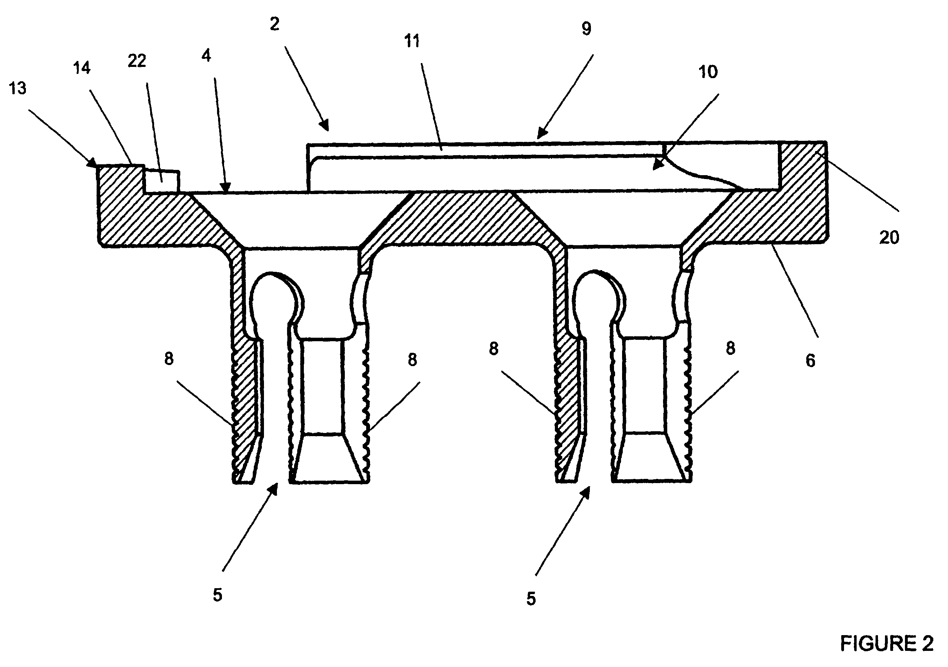

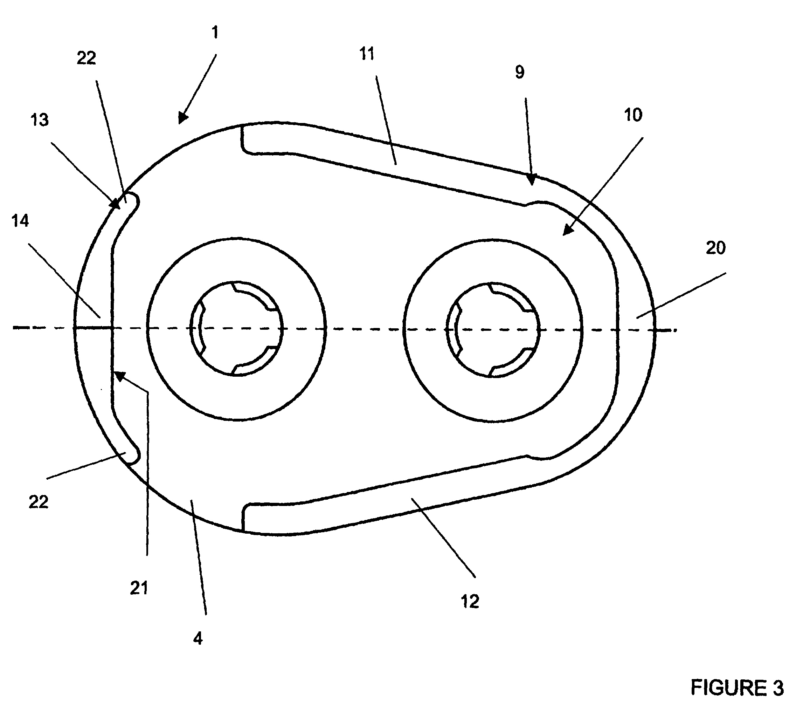

On a montré en figures 1 à 3 une embase métallique 1 d'une prothèse, et notamment d'une prothèse glénoïdienne de l'omoplate comportant un dispositif de fixation 2 pour la retenue d'un insert 3 en matière plastique.

L'embase métallique 1 est constituée d'un plateau 4 disposé dans un plan horizontal et solidaire d'au moins une cheville d'ancrage 5 s'étendant perpendiculairement à la face d'appui 6 dudit plateau comme cela est décrit dans le brevet français n° 2 741 796 au nom du demandeur.

Chaque cheville d'ancrage 5 reçoit une vis 7 qui coopère avec un écrou, non représenté, permettant la déformation plastique des ailettes 8 lors de la fixation de l'embase métallique 1 contre l'os d'un patient.

Le plateau 4 de l'embase métallique 1 est solidaire à l'opposé de la face d'appui 6 d'un rebord périphérique 9 qui s'étend verticalement par rapport audit plateau. Le rebord périphérique 9 suit le pourtour extérieur de l'embase 1 pour présenter un profil en V et délimiter une cuvette 10 ouverte vers le haut.

Le rebord périphérique 9 est constitué de deux tronçons opposés 11 et 12 qui sont reliés entre eux par l'intermédiaire d'une portion 20 à profil courbe délimitant une butée dans la partie haute de l'embase métallique.

Les tronçons 11 et 12 sont inclinés suivant le profil du V afin de se refermer en direction du haut de l'embase métallique, tandis qu'ils présentent une large ouverture du côté du bas de ladite embase.

Chaque tronçon 11, 12 présente un profil en queue d'aronde dont le pan incliné est rentrant et dirigé en direction du plateau 4 de l'embase métallique 1.

La cuvette 10 est également délimitée à l'opposé de la portion à profil courbe 20 par un bord périphérique 13 également à profil courbe situé du côté de la base la plus ouverte du V du rebord 9.

Le bord périphérique 13 s'étend verticalement au-dessus du plateau 4 et suit le pourtour extérieur de l'embase métallique 1.

Le bord périphérique 13 à profil courbe comporte du côté de la cuvette 10, une butée 14 qui est disposée à l'opposé de la portion à profil courbe 20 reliant les tronçons 11 et 12.

Le bord périphérique 13 présente, à partir du plateau 4, une hauteur qui est inférieure à celle prévue pour les tronçons 11, 12 et la portion courbe 20.

La butée 14 s'étend verticalement à partir du plateau 4 pour constituer une zone d'appui constante sur toute sa largeur et sa hauteur.

La butée 14 comporte une portion droite 21 délimitée à chaque extrémité par une portion courbe 22 afin de récupérer le profil du bord périphérique 13.

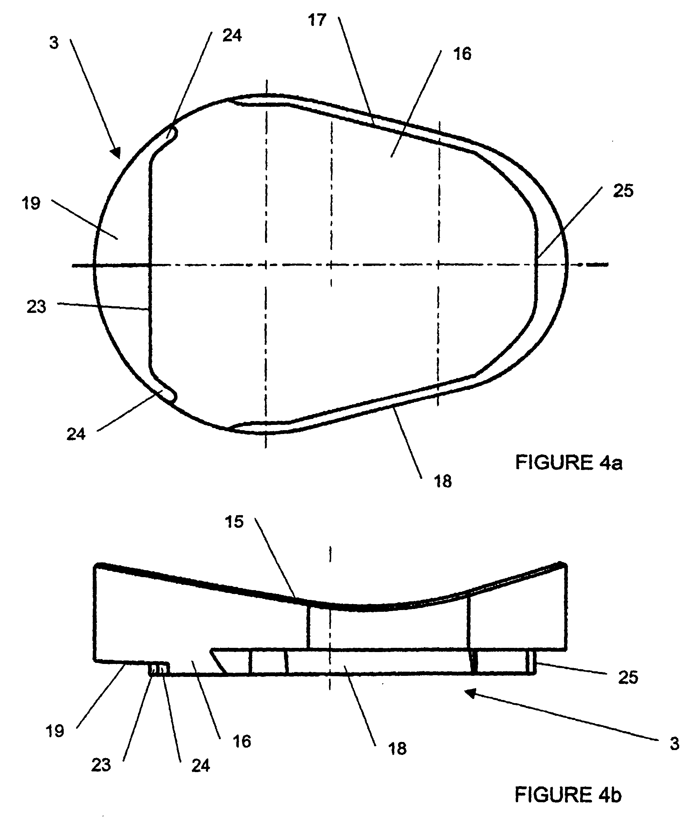

On a montré en figures 4a et 4b l'insert 3 en matière plastique qui comporte à l'opposé de sa surface articulaire 15 une partie en saillie 16 présentant un profil complémentaire à celui de la cuvette 10 délimitée par le rebord périphérique 9 et le bord périphérique 13.

La partie en saillie 16 comporte des bords périphériques opposés 17, 18 qui sont inclinés pour coopérer respectivement avec les tronçons 11, 12 du rebord périphérique 9 en forme de V de l'embase métallique 1.

Chaque bord périphérique opposé 17, 18 de la partie en saillie 16 présente un profil en queue d'aronde afin de coopérer avec le profil des tronçons inclinés 11, 12 du rebord périphérique 9.

Ainsi, la partie en saillie 16 présente un profil en forme de V semblable à celui délimité par le rebord périphérique 9 de l'embase métallique 1.

La partie en saillie 16 comporte du côté de la base la plus ouverte du V une échancrure 19 qui présente un profil complémentaire à celui du bord périphérique 13 et de la butée 14.

En effet, la partie en saillie 16 comporte du côté de l'échancrure 19 une portion droite 23 disposée dans un même plan que celle 21 de la butée 14.

La portion droite 23 de la partie en saillie 16 se termine à chaque extrémité par un logement incliné 24 de même profil que les portions courbes 22 de la butée 14.

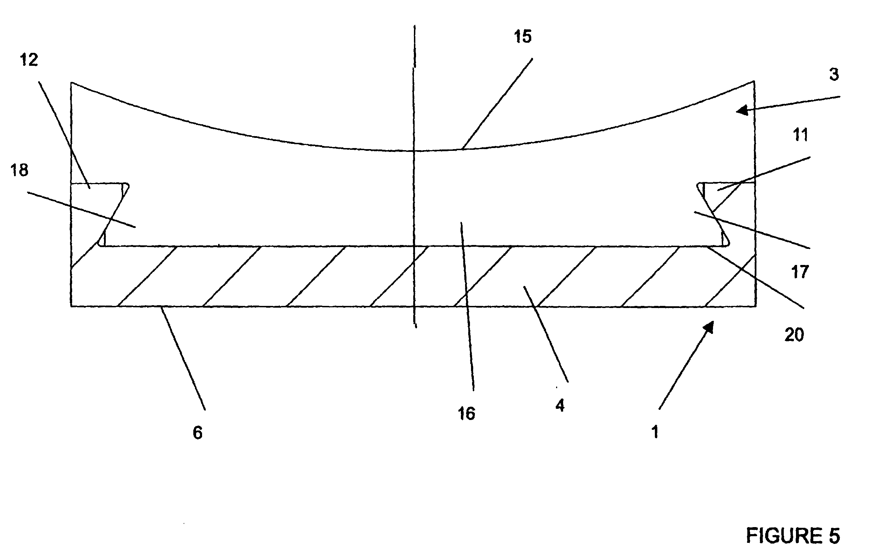

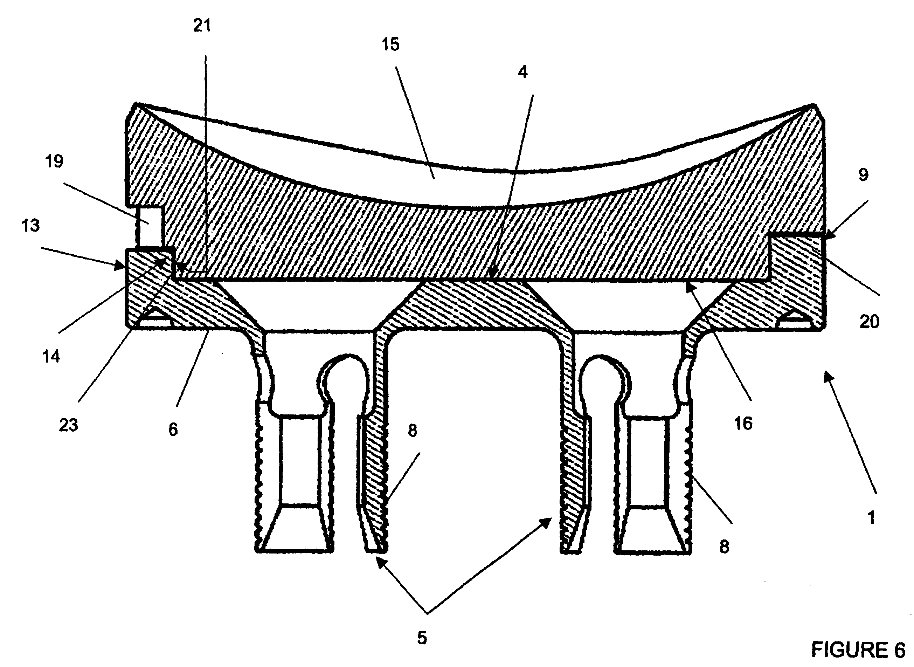

On a montré en figures 5 et 6 l'insert 3 qui est introduit sur l'embase métallique 1 de manière que ses bords 17, 18 coopèrent avec les tronçons 11, 12 jusqu'à ce que la partie en saillie 16, et plus particulièrement le bord 25 opposé à l'échancrure 19, vienne en butée contre la portion 20 du rebord périphérique 9.

Ensuite, le blocage de l'insert 3 est réalisé par une légère déformation élastique de ce dernier, afin que les portions 23 et 24 de la partie en saillie 16 vienne en appui contre la butée 14 du bord périphérique 13 de l'embase 1 (figure 6).

On remarque que l'échancrure 19 ménagée dans la partie en saillie 16 se trouve au-dessus du bord périphérique 13 et de la butée 14 lorsque l'insert 3 est engagé dans la cuvette 10 de l'embase métallique 1.

L'échancrure 19 permet l'introduction d'un outil pour déformer élastiquement la partie en saillie 16, et plus particulièrement la portion droite 23, pour permettre l'extraction de l'insert 3 de la cuvette 10 de l'embase métallique 1.

On constate que l'insert 3 est retenu sur l'embase 1 dès que sa partie en saillie 16 est introduite intégralement dans la cuvette 10.

On note que la fixation de l'insert 3 dans l'embase métallique 1 est obtenue suivant quatre directions à savoir :
- Deux mouvements latéraux portés par les axes principaux de l'embase,
- Une rotation autour de l'axe vertical,
- Et une contrainte à l'arrachement.

Cette retenue combinée avec la portion 20 à profil courbe du rebord 9 permet de répondre aux problèmes des efforts à l'arrachement qui sont situés dans la partie haute de l'embase métallique 1.

En effet, dans une glène de l'épaule, les contraintes les plus fortes poussent l'insert 3 à monter en direction de la portion 20. Le rebord 9 en forme de V s'oppose à cette contrainte et empêche l'insert 3 de monter.

Par contre, la butée 14 empêche le mouvement inverse de descente de l'insert 3. La butée 14 permet de bloquer l'insert 3 au niveau où il existe le moins d'effort.

## Revendications

1. Dispositif pour la fixation d'un insert (3) en matière plastique sur une embase métallique (1), comprenant:
• une embase métallique (1) pourvue d'une cuvette (10) qui est délimitée par un rebord périphérique (9) en forme de V possédant des tronçons opposés et inclinés (11, 12) qui sont reliés par une portion courbe (20), et par un bord périphérique (13) solidaire d'une butée (14) positionnée du côté de la base la plus ouverte du V du rebord (9),
• et un insert (3) qui comporte à l'opposé de sa surface articulaire (15) une partie en saillie (16) présentant un profil complémentaire à celui de la cuvette (10) pour coopérer avec les tronçons inclinés (11, 12) et venir en appui contre la butée (14).

2. Dispositif de fixation suivant la revendication 1, **caractérisé en ce que** le rebord périphérique (9) comprend des tronçons inclinés (11, 12) comportant un profil en forme de queue d'aronde.

3. Dispositif de fixation suivant la revendication 1, **caractérisé en ce que** la butée (14) s'étend verticalement à partir du plateau (4) de l'embase métallique (1).

4. Dispositif de fixation suivant la revendication 1, **caractérisé en ce que** la butée (14) comporte une portion droite (21) délimitée à chaque extrémité par une portion courbe (22).

5. Dispositif de fixation suivant la revendication 1, **caractérisé en ce que la** partie en saillie (16) de l'insert (3) comporte des bords périphériques opposés (17, 18) qui sont inclinés pour coopérer respectivement avec les tronçons (11, 12) du rebord périphérique (9) en forme de V de l'embase métallique (1).

6. Dispositif de fixation suivant la revendication 5, **caractérisé en ce que** les bords périphériques opposés (17, 18) de la partie en saillie (16) sont en forme de queue d'aronde afin de coopérer avec le profil des tronçons inclinés (11, 12).

7. Dispositif de fixation suivant la revendication 1, **caractérisé en ce que** la partie en saillie (16) de l'insert (3) comporte une échancrure (19) qui présente un profil complémentaire à celui du bord périphérique (13) et de la butée (14).

8. Dispositif de fixation suivant la revendication 1, **caractérisé en ce que** la partie en saillie (16) comporte du côté de l'échancrure (19) une portion droite (23) se terminant à chaque extrémité par une portion courbe (24).

9. Prothèse d'épaule comportant un dispositif de fixation d'un insert (3) en matière plastique sur une embase métallique (1) l'embase métallique (1) est pourvue d'une cuvette (10) qui est délimitée par un rebord périphérique (9) en forme de V possédant des tronçons opposés et inclinés (11, 12) qui sont reliés par une portion courbe (20) et par un bord périphérique (13) solidaire d'une butée (14) positionnée du côté de la base la plus ouverte du V du rebord (9), tandis que l'insert (3) comporte à l'opposé de sa surface articulaire (15) une partie en saillie (16) présentant un profil complémentaire à celui de la cuvette (10) pour coopérer avec les tronçons tel que défini dans la revendication 1.

10. Prothèse d'épaule suivant la revendication 9, **caractérisée en ce que** le rebord périphérique (9) comprend des tronçons inclinés (11, 12) comportant un profil en forme de queue d'aronde.

11. Prothèse d'épaule suivant la revendication 9, **caractérisée en ce que** la butée (14) s'étend à partir du plateau (4) de l'embase métallique (1) et sur toute la hauteur du bord périphérique (13).

12. Prothèse d'épaule suivant la revendication 9, **caractérisée en ce que** la butée (14) comporte une portion droite (21) délimitée à chaque extrémité par une portion courbe (22).

13. Prothèse d'épaule suivant la revendication 9, **caractérisée en ce que** la partie en saillie (16) de l'insert (3) comporte des bords périphériques opposés (17, 18) qui sont inclinés pour coopérer respectivement avec les tronçons (11, 12) du rebord périphérique (9) en forme de V de l'embase métallique (1 ).

14. Prothèse d'épaule suivant la revendication 13, **caractérisée en ce que** les bords périphériques opposés (17, 18) de la partie en saillie (16) sont en forme de queue d'aronde afin de coopérer avec le profil des tronçons inclinés (11, 12).

15. Prothèse d'épaule suivant la revendication 9, **caractérisée en ce que** la partie en saillie (16) de l'insert (3) comporte une échancrure (19) qui présente un profil complémentaire à celui du bord périphérique (13) et de la butée (14).

16. Prothèse d'épaule suivant la revendication 9, **caractérisée en ce que** la partie en saillie (16) comporte du côté de l'échancrure (19) une portion droite (23) se terminant à chaque extrémité par une portion courbe (24).

## Patentansprüche

1. Vorrichtung zur Befestigung eines Einsatzes (3) aus Kunststoff auf einem Metallsockel (1), die Folgendes umfasst:
• einen Metallsockel (1), der mit einer Wanne (10) versehen ist, die von einem peripheren Rand (9) in V-Form abgegrenzt ist, die einander gegenüberliegende und schräge Abschnitte (11, 12) besitzt, welche durch einen gebogenen Teil (20) verbunden sind und durch einen peripheren Rand (13), der fest mit einem Anschlag (14) verbunden ist, der auf der Seite der weiter geöffneten Basis des V des Rands (9) angeordnet ist,
• und einen Einsatz (3), der seiner gelenkigen Fläche (15) gegenüber einen vorstehenden Teil (16) umfasst, der ein ergänzendes Profil zu dem der Wanne (10) hat, um mit den schrägen Abschnitten (11, 12) zusammenzuarbeiten und gegen den Anschlag (14) zur Auflage zu kommen.

2. Vorrichtung zur Befestigung nach Anspruch 1, **dadurch gekennzeichnet, dass** der periphere Rand (9) schräge Abschnitte (11, 12) umfasst, die ein Profil in Schwalbenschwanzform aufweisen.

3. Vorrichtung zur Befestigung nach Anspruch 1, **dadurch gekennzeichnet, dass** der Anschlag (14) sich ausgehend von der Platte (4) des Metallsockels (1) senkrecht erstreckt.

4. Vorrichtung zur Befestigung nach Anspruch 1, **dadurch gekennzeichnet, dass** der Anschlag (14) einen geraden Teil (21) umfasst, der an jedem Ende von einem gebogenen Teil (22) abgegrenzt wird.

5. Vorrichtung zur Befestigung nach Anspruch 1, **dadurch gekennzeichnet, dass** der vorstehende Teil (16) des Einsatzes (3) einander gegenüberliegende periphere Ränder (17, 18) umfasst, die schräg sind, um jeweils mit den Abschnitten (11, 12) des peripheren Rands (9) in V-Form des Metallsockels (1) zusammenzuarbeiten.

6. Vorrichtung zur Befestigung nach Anspruch 5, **dadurch gekennzeichnet, dass** die einander gegenüberliegenden peripheren Ränder (17, 18) des vorstehenden Teils (16) Schwalbenschwanzform haben, um mit dem Profil der schrägen Abschnitte (11, 12) zusammenzuarbeiten.

7. Vorrichtung zur Befestigung nach Anspruch 1, **dadurch gekennzeichnet, dass** der vorstehende Teil (16) des Einsatzes (3) einen Ausschnitt (19) umfasst, der ein ergänzendes Profil zu dem des peripheren Rands (13) und des Anschlags (14) aufweist.

8. Vorrichtung zur Befestigung nach Anspruch 1, **dadurch gekennzeichnet, dass** der vorstehende Teil (16) auf der Seite des Ausschnitts (19) einen geraden Teil (23) umfasst, der an jedem Ende in einem gebogenen Teil (24) endet.

9. Schulterprothese mit einer Vorrichtung zur Befestigung eines Einsatzes (3) aus Kunststoff auf einem Metallsockel (1), wobei der Metallsockel (1) mit einer Wanne (10) versehen ist, die von einem peripheren Rand (9) in V-Form abgegrenzt wird, die einander gegenüberliegende und schräge Abschnitte (11, 12) besitzt, die durch einen gebogenen Teil (20) und durch einen peripheren Rand (13) fest verbunden mit einem Anschlag (14), der auf der Seite der weiter geöffneten Basis des V des Rands(9) angeordnet ist, verbunden sind, während der Einsatz (3) auf der seiner gelenkigen Fläche (15) gegenüberliegenden Seite einen vorstehenden Teil (16) umfasst, der ein ergänzendes Profil zu dem der Wanne (10) aufweist, um mit den Abschnitten wie in Anspruch 1 definiert zusammenzuarbeiten.

10. Schulterprothese nach Anspruch 9, **dadurch gekennzeichnet, dass** der periphere Rand (9) schräge Abschnitte (11, 12) umfasst, die ein Profil in Schwalbenschwanzform aufweisen.

11. Schulterprothese nach Anspruch 9, **dadurch gekennzeichnet, dass** der Anschlag (14) sich ausgehend von der Platte (4) des Metallsockels (1) und über die ganze Höhe des peripheren Rands (13) erstreckt.

12. Schulterprothese nach Anspruch 9, **dadurch gekennzeichnet, dass** der Anschlag (14) einen geraden Teil (21) umfasst, der an jedem Ende von einem gebogenen Teil (22) abgegrenzt ist.

13. Schulterprothese nach Anspruch 9, **dadurch gekennzeichnet, dass** der vorstehende Teil (16) des Einsatzes (3) einander gegenüberliegende periphere Ränder (17, 18) umfasst, die schräg sind, um jeweils mit den Abschnitten (11, 12) des peripheren Rands (9) in V-Form des Metallsockels (1) zusammenzuarbeiten.

14. Schulterprothese nach Anspruch 13, **dadurch gekennzeichnet, dass** die einander gegenüberliegenden peripheren Ränder (17, 18) des vorstehenden Teils (16) Schwalbenschwanzform haben, um mit dem Profil der schrägen Abschnitte (11, 12) zusammenzuarbeiten.

15. Schulterprothese nach Anspruch 9, **dadurch gekennzeichnet, dass** der vorstehende Teil (16) des Einsatzes (3) einen Ausschnitt (19) umfasst, der ein zum peripheren Rand (13) des Anschlags (14) ergänzendes Profil aufweist.

16. Schulterprothese nach Anspruch 9, **dadurch gekennzeichnet, dass** der vorstehende Teil (16) auf der Seite des Ausschnitts (19) einen geraden Teil (23) umfasst, der an jedem Ende in einem gebogenen Teil (24) endet.

## Claims

1. Device for fixing a plastic insert (3) on a metal seat (1), comprising
• a metal seat (1) provided with a cell (10) which is delimited by a peripheral V-shaped flange (9), with opposite and inclined portions (11, 12) which are connected via a curved portion (20), and by a peripheral edge (13) integral with an abutment (14) which is positioned towards the more open base of the V of the flange (9),
• and an insert (3) which comprises, opposite its articular surface (15), a projecting part (16) with a profile complementing that of the cell (10) so as to cooperate with the inclined portions (11, 12) and come to bear against the abutment (14).

2. Fixing device according to Claim 1, **characterized in that** the peripheral flange (9) comprises inclined portions (11, 12) which have a dovetail-shaped profile.

3. Fixing device according to Claim 1, **characterized in that** the abutment (14) extends vertically from the plateau (4) of the metal seat (1).

4. Fixing device according to Claim 1, **characterized in that** the abutment (14) comprises a straight portion (21) delimited at each end by a curved portion (22).

5. Fixing device according to Claim 1, **characterized in that** the projecting part (16) of the insert (3) comprises opposite peripheral edges (17, 18) which are inclined in order to cooperate respectively with the portions (11, 12) of the peripheral V-shaped flange (9) of the metal seat (1).

6. Fixing device according to Claim 5, **characterized in that** the opposite peripheral edges (17, 18) of the projecting part (16) are dovetail-shaped in order to cooperate with the profile of the inclined portions (11, 12).

7. Fixing device according to Claim 1, **characterized in that** the projecting part (16) of the insert (3) comprises an indent (19) with a profile complementing that of the peripheral edge (13) and of the abutment (14).

8. Fixing device according to Claim 1, **characterized in that** the projecting part (16) comprises, towards the indent (19), a straight portion (23) terminating at each end in a curved portion (24).

9. Shoulder prosthesis comprising a device for fixing a plastic insert (3) on a metal seat (1), the metal seat (1) being provided with a cell (10) which is delimited by a peripheral V-shaped flange (9), with opposite and inclined portions (11, 12) which are connected via a curved portion (20), and by a peripheral edge (13) integral with an abutment (14) which is positioned towards the more open base of the V of the flange (9), while the insert (3) comprises, opposite its articular surface (15), a projecting part (16) with a profile complementing that of the cell (10) so as to cooperate with the portions, as defined in Claim 1.

10. Shoulder prosthesis according to Claim 9, **characterized in that** the peripheral flange (9) comprises inclined portions (11, 12) with a dovetail-shaped profile.

11. Shoulder prosthesis according to Claim 9, **characterized in that** the abutment (14) extends from the plateau (4) of the metal seat (1) and over the entire height of the peripheral edge (13).

12. Shoulder prosthesis according to Claim 9, **characterized in that** the abutment (14) comprises a straight portion (21) delimited at each end by a curved portion (22).

13. Shoulder prosthesis according to Claim 9, **characterized in that** the projecting part (16) of the insert (3) comprises opposite peripheral edges (17, 18) which are inclined in order to cooperate respectively with the portions (11, 12) of the peripheral V-shaped flange (9) of the metal seat (1).

14. Shoulder prosthesis according to Claim 13, **characterized in that** the opposite peripheral edges (17, 18) of the projecting part (16) are dovetail-shaped in order to cooperate with the profile of the inclined portions (11, 12).

15. Shoulder prosthesis according to Claim 9, **characterized in that** the projecting part (16) of the insert (3) comprises an indent (19) with a profile complementing that of the peripheral edge (13) and of the abutment (14).

16. Shoulder prosthesis according to Claim 9, **characterized in that** the projecting part (16) comprises, towards the indent (19), a straight portion (23) terminating at each end in a curved portion (24).
